# EUROPEAN PATENT APPLICATION

(11) **EP 4 681 644 A1**
(43) Date of publication of application: **21.01.2026**
(21) Application number: 24770582.5
(22) Date of filing: 01.03.2024
(51) Int. Cl.: A61B 5/327

(54) **BIOLOGICAL-SIGNAL-PROCESSING SYSTEM, SIGNAL-PROCESSING DEVICE, COMPUTER PROGRAM, AND METHOD FOR GENERATING BIOLOGICAL SIGNAL**

(30) Priority: 13.03.2023 JP 2023038749
(71) Applicant: National University Corporation Kobe University, Kobe-shi, Hyogo 657-8501 (JP); The University of Osaka, Osaka 565-0871 (JP)
(72) Inventor: IZUMI, Shintaro, Kobe-shi, Hyogo 657-8501 (JP); ARAKI, Teppei, Suita-shi, Osaka 565-0871 (JP); MURASE, Sho, Suita-shi, Osaka 565-0871 (JP)
(74) Representative: RGTH
(86) International application number: PCT/JP2024/007846
(87) International publication number: WO 2024/190471

(57) **Abstract**

Even in another electrode arrangement different from a predetermined electrode arrangement, a necessary multiple-channel biological signal can be obtained. A biological signal processing system of this disclosure includes: m electrodes attached in a first arrangement to a living body; and a signal processing device configured to execute a process of inputting a first biological signal of p channels acquired from the m electrodes, into a generative model, and outputting a second biological signal of q channels, from the generative model. The second biological signal of the q channels is a signal corresponding to signals obtained from n (n is an integer of 3 or larger) electrodes attached in a second arrangement to the living body. The second arrangement is an electrode arrangement different from the first arrangement in at least one of the number of electrodes and an attachment position. The generative model is configured to output the second biological signal of the q channels when the first biological signal of the p channels has been inputted.

## Description

### TECHNICAL FIELD

The present disclosure relates to a biological signal processing system, a signal processing device, a computer program, and a biological signal generation method.

### BACKGROUND ART

PATENT LITERATURE 1 discloses a biological signal measurement device. The device of PATENT LITERATURE 1 measures temporal change in potential such as electrical activity in a living body, by using an electrode. PATENT LITERATURE 1 discloses that the device can be applied to an electroencephalograph and, in addition, can be applied to an electrocardiograph and the like.

### CITATION LIST

### [PATENT LITERATURE]

PATENT LITERATURE 1: International Publication No. WO2017/122379

### SUMMARY OF INVENTION

Examples of measurement of a biological signal include measurement for obtaining a standard 12-lead electrocardiogram. The standard 12-lead electrocardiogram is composed of 12 electrocardiograms (electrocardiograms of 12 channels). In order to record a standard 12-lead electrocardiogram, a total of ten electrodes, i.e., four limb electrodes and six chest electrodes, are used. In order to obtain an appropriate standard 12-lead electrocardiogram, it is necessary to accurately attach a predetermined number (ten) of electrodes at predetermined positions in a living body.

Thus, when a standard 12-lead electrocardiogram or another necessary multiple-channel biological signal is to be recorded, a predetermined number of electrodes need to be arranged at predetermined positions in a living body. To date, it is possible to obtain a necessary multiple-channel biological signal with a predetermined electrode arrangement, but not with another electrode arrangement.

However, requiring a predetermined electrode arrangement in which a predetermined number of electrodes are arranged at predetermined positions in a living body may lead to decrease in convenience. In addition, it may be also difficult to obtain the predetermined electrode arrangement. Therefore, it is desired to make it possible to obtain a necessary multiple-channel biological signal even with another electrode arrangement different from the predetermined electrode arrangement.

An aspect of the present disclosure is a biological signal processing system. The biological signal processing system of the disclosure includes: m (m is an integer of 3 or larger) electrodes attached in a first arrangement to a living body; and a signal processing device configured to execute a process of inputting a first biological signal of p (p is an integer of 2 or larger) channels acquired from the m electrodes, into a generative model, and of outputting a second biological signal of q (q is an integer of 2 or larger) channels, from the generative model. The second biological signal of the q channels is a signal corresponding to signals obtained from n (n is an integer of 3 or larger) electrodes attached in a second arrangement to the living body. The second arrangement is an electrode arrangement different from the first arrangement in at least one of the number of electrodes and an attachment position. The generative model is configured to output the second biological signal of the q channels when the first biological signal of the p channels has been inputted.

Another aspect of the present disclosure includes a signal processing device, a biological signal generation method, or a computer program. Further details will be described as an embodiment described later.

### BRIEF DESCRIPTION OF DRAWINGS

FIG. 1 is a configuration diagram of a biological signal processing system.
FIG. 2 is a cross-sectional view along an A-Aline of an electrode device.
FIG. 3 is a diagram illustrating a state where electrodes are affixed using the electrode device according to the embodiment.
FIG. 4 is a diagram illustrating the positions of electrodes for a standard 12-lead electrocardiogram.
FIG. 5 is a flowchart showing a procedure of a process performed in a signal processing device.
FIG. 6 shows waveforms each indicating a first biological signal.
FIG. 7 is a diagram illustrating a learning phase and an inference phase of a generative model.
FIG. 8 shows waveforms each indicating a second biological signal.

### DESCRIPTION OF EMBODIMENTS

### <1. Outline of biological signal processing system, signal processing device, biological signal generation method, and computer program>

(1) A system according to an embodiment can be a biological signal processing system. The biological signal processing system can include: m (m is an integer of 3 or larger) electrodes attached in a first arrangement to a living body; and a signal processing device configured to execute a process of inputting a first biological signal of p (p is an integer of 2 or larger) channels acquired from the m electrodes, into a generative model, and of outputting a second biological signal of q (q is an integer of 2 or larger) channels, from the generative model. The second biological signal of the q channels can be a signal corresponding to signals obtained from n (n is an integer of 3 or larger) electrodes attached in a second arrangement to the living body. The second arrangement can be an electrode arrangement different from the first arrangement in at least one of the number of electrodes and an attachment position. The generative model can be configured to output the second biological signal of the q channels when the first biological signal of the p channels has been inputted.
   In the biological signal processing system according to the embodiment, the second biological signal corresponding to the signals obtained from the electrodes in the second arrangement can be generated from the first biological signal acquired from the electrodes in the first arrangement different from the second arrangement. That is, even in an electrode arrangement of the first arrangement different from the predetermined second arrangement, the necessary second biological signal can be obtained.
(2) Preferably, the m electrodes attached in the first arrangement to the living body are provided on a single base in a state where a relative positional relationship between the m electrodes is fixed. When the relative positional relationship between the electrodes is fixed, attachment of the electrodes becomes easy.
(3) Preferably, the generative model is a learned model having been subjected to machine learning so as to output the second biological signal of the q channels when the first biological signal of the p channels has been inputted.
(4) Preferably, the second biological signal of the q channels is a signal of 12 channels in a standard 12-lead electrocardiogram or a signal of less than 12 channels included in the standard 12-lead electrocardiogram.
(5) Preferably, the m electrodes from which the first biological signal of the p channels is acquired are all attached at a chest center of the living body.
(6) A device according to the embodiment can be a signal processing device configured to execute signal processing. The signal processing can include: acquiring a first biological signal of p (p is an integer of 2 or larger) channels from m (m is an integer of 3 or larger) electrodes attached in a first arrangement to a living body; and inputting the first biological signal of the p channels into a generative model, and outputting a second biological signal of q (q is an integer of 2 or larger) channels, from the generative model. The second biological signal of the q channels can be a signal corresponding to signals obtained from n (n is an integer of 3 or larger) electrodes attached in a second arrangement to the living body. The second arrangement can be an electrode arrangement different from the first arrangement in at least one of the number of electrodes and an attachment position. The generative model can be configured to output the second biological signal of the q channels when the first biological signal of the p channels has been inputted.
(7) A method according to the embodiment can be a biological signal generation method. The generation method can include: acquiring a first biological signal of p (p is an integer of 2 or larger) channels from m (m is an integer of 3 or larger) electrodes attached in a first arrangement to a living body; and inputting the first biological signal of the p channels into a generative model, and outputting a second biological signal of q (q is an integer of 2 or larger) channels, from the generative model. The second biological signal of the q channels can be a signal corresponding to signals obtained from n (n is an integer of 3 or larger) electrodes attached in a second arrangement to the living body. The second arrangement can be an electrode arrangement different from the first arrangement in at least one of the number of electrodes and an attachment position. The generative model can be configured to output the second biological signal of the q channels when the first biological signal of the p channels has been inputted.
(8) A computer program according to the embodiment can be a computer program configured to cause a computer to execute signal processing. The signal processing can include: acquiring a first biological signal of p (p is an integer of 2 or larger) channels from m (m is an integer of 3 or larger) electrodes attached in a first arrangement to a living body; and inputting the first biological signal of the p channels into a generative model, and outputting a second biological signal of q (q is an integer of 2 or larger) channels, from the generative model. The second biological signal of the q channels can be a signal corresponding to signals obtained from n (n is an integer of 3 or larger) electrodes attached in a second arrangement to the living body. The second arrangement can be an electrode arrangement different from the first arrangement in at least one of the number of electrodes and an attachment position. The generative model can be configured to output the second biological signal of the q channels when the first biological signal of the p channels has been inputted.

The computer program can be stored in a non-transitory computer-readable storage medium.

### <2. Example of outline of biological signal processing system, signal processing device, computer program, and biological signal generation method>

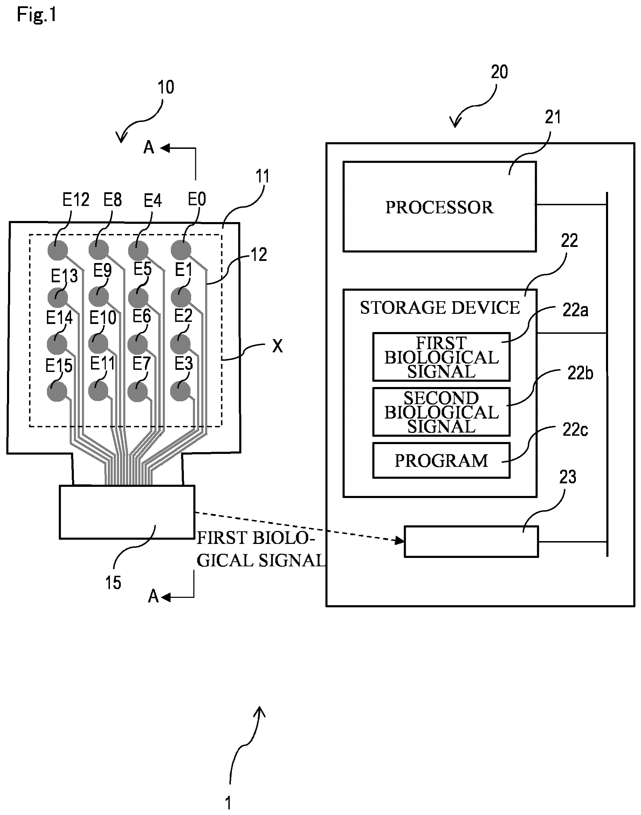

FIG. 1 shows a biological signal processing system 1 according to an embodiment. The system 1 includes an electrode device 10 and a signal processing device 20. The electrode device 10 measures a first biological signal. The signal processing device 20 reconstructs, from the first biological signal, a second biological signal different from the first biological signal.

The electrode device 10 is attached to a surface of a living body in order to measure the first biological signal. The first biological signal is a signal measured by the electrode device 10 according to the embodiment. The living body is a person, for example. The biological signal is the body surface potential of the living body, for example. The body surface potential is a minute potential (several tens of microvolts to several tens of millivolts) generated through activities of the heart or other muscles, for example.

The electrode device 10 includes multiple electrodes E0 to E15. Here, the "multiple electrodes E0 to E15" are also referred to as "m electrodes E0 to E15". Preferably, m is an integer of 3 or larger. For body surface potential measurement, each electrode E0 to E15 is attached so as to be in contact with the body surface. The electrode device 10 is attached to the body surface such that each electrode E0 to 15 is in contact with the body surface.

The electrode device 10 includes a sheet 11 as a base for supporting the electrodes E0 to E15. The sheet 11 is formed from a thin film-like synthetic resin or another material, for example. Preferably, the sheet 11 has flexibility. When the sheet 11 has flexibility, the sheet 11 can be affixed along the shape of the body surface. Preferably, the sheet 11 is stretchable in the planar direction. Preferably, the sheet 11 is transparent or translucent.

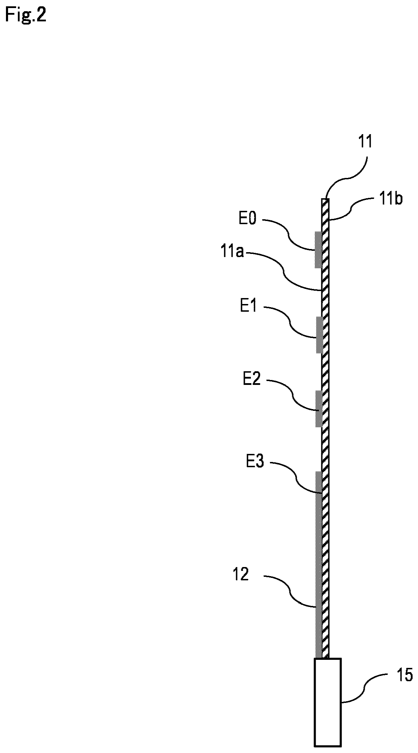

As shown in FIG. 2, the sheet 11 has a first surface 11a, and a second surface 11b which is the opposite surface to the first surface 11a. The first surface 11a is the surface that is affixed to the body surface of the living body. The first surface 11a has adhesiveness so as to be affixed to the body surface. The sheet 11 itself may have the adhesiveness, or the adhesiveness may be obtained by applying an adhesive to the first surface 11a, or by performing another surface treatment for causing adhesiveness.

The m electrodes E0 to E15 are provided on the first surface 11a. The m electrodes E0 to E15 are provided so as to be exposed on the first surface 11a. Therefore, when the sheet 11 has been affixed to the body surface such that the first surface 11a is in contact with the body surface, the electrodes E0 to E15 are in in contact with the skin which is the body surface. Each electrode E0 to E15 measures the body surface potential at the position where the electrode E0 to E15 is affixed.

In the electrode device 10 of the embodiment, all the electrodes E0 to E15 are provided in an integrated manner on a single sheet 11, and thus, the multiple electrodes E0 to E15 can be attached to the person, through one step of affixing the sheet 11. Therefore, the work of attaching the electrode device 10 is easy.

In the electrode device 10 of the embodiment, all the electrodes E0 to E15 are provided in a position-fixed manner on the sheet 11. As a result, the relative positional relationship between the electrodes E0 to E15 is restricted and fixed by the single sheet 11 (base). That the relative positional relationship between the electrodes E0 to E15 is in a fixed state is advantageous for accurately performing reconstruction of the second biological signal.

The first surface 11a of the sheet 11 is also provided with a wiring 12. The wiring 12 extends from each of the electrodes E0 to E15 to a communication device 15 mounted to the sheet 11. The wiring 12 is connected to the communication device 15 via a connector (not shown).

The communication device 15 transmits the first biological signal measured by each of the electrodes E0 to E15 to the signal processing device 20. For example, the communication device 15 includes a signal processing circuit and a wireless circuit. The signal processing circuit includes: an amplifier that amplifies the first biological signal measured by each electrode E0 to E15; an AD converter that converts the amplified signal into a digital signal; and the like, for example. The wireless circuit wirelessly transmits a radio signal obtained by modulating the digital signal, to the signal processing device 20. The communication device 15 may transmit the first biological signal to the signal processing device 20 through wired transmission. The signal processing device 20 receives the first biological signal transmitted from the communication device 15, and executes a process described later by using the first biological signal, to generate the second biological signal.

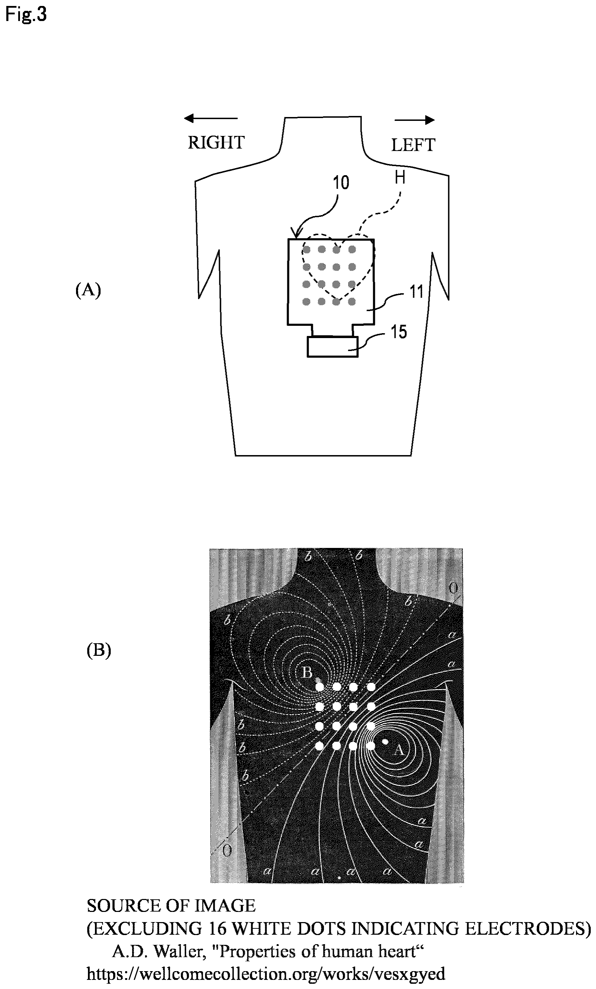

As shown in FIG. 3(A), as an example, the electrode device 10 is attached to a person such that the electrodes E0 to E15 are positioned at the center of the chest of the person. When the electrodes E0 to E15 are attached at the chest center, many of the electrodes E0 to E15 are present, on the body surface, at positions overlapping a heart H, which is advantageous. At the body surface (vicinity of chest center) near the heart H, there is large change in the body surface potential as described later. Therefore, arranging many integrated electrodes E0 to E15 at the body surface (vicinity of chest center) near the heart H allows obtainment of much information about the body surface potential, which is advantageous.

Here, the chest of a person refers to the area, in the body surface on the anterior side of the person, where the sternum and ribs are present in the body. The chest center refers to the position approximately at the center in the left right direction of the chest. Since the heart is at (slightly to the left of) the chest center, the chest center is at a position in the vicinity of the heart.

FIG. 3(B) shows an example of the body surface potential distribution in the upper body including the chest of a person. In FIG. 3(B), 16 white round dots indicate 16 electrodes E0 to E15 attached at the chest center. In FIG. 3(B), white lines drawn on the human body indicate that the positions of the white lines are at the same potential. In FIG. 3(B), A indicates either one of the position having the highest potential and the position having the lowest potential, and B indicates the other. The potentials of A and B respectively occur at both ends of the heart. As shown in FIG. 3(B), between A and B, the white lines are densely present and potential change is large.

As shown in FIG. 3(B), most of the values of potentials present on the body surface of the person are distributed at the chest center (approximately corresponding to the area where the 16 white round dots are present in FIG. 3(B)). That is, almost all the white lines indicating the same potential in FIG. 3(B) pass through the chest center. The distribution of potentials in the area other than the chest center is determined according to the distribution of the white lines passing through the vicinity of the chest center. Therefore, information of potentials at multiple points in the area of the chest center serves as useful information for estimating the potential at a body surface position other than the chest center. Therefore, if the first biological signal obtained by attaching the electrode device 10 to the chest center is used, the second biological signal at another position can be obtained.

In the embodiment, as an example, the signal processing device 20 generates the second biological signal corresponding to a standard 12-lead electrocardiogram, by using the first biological signal. In general, when a standard 12-lead electrocardiogram is to be obtained, a total of ten electrodes, i.e., four limb electrodes R, L, F, N and six chest electrodes C1, C2, C3, C4, C5, C6, are used, to measure the potential at the attachment position of each electrode.

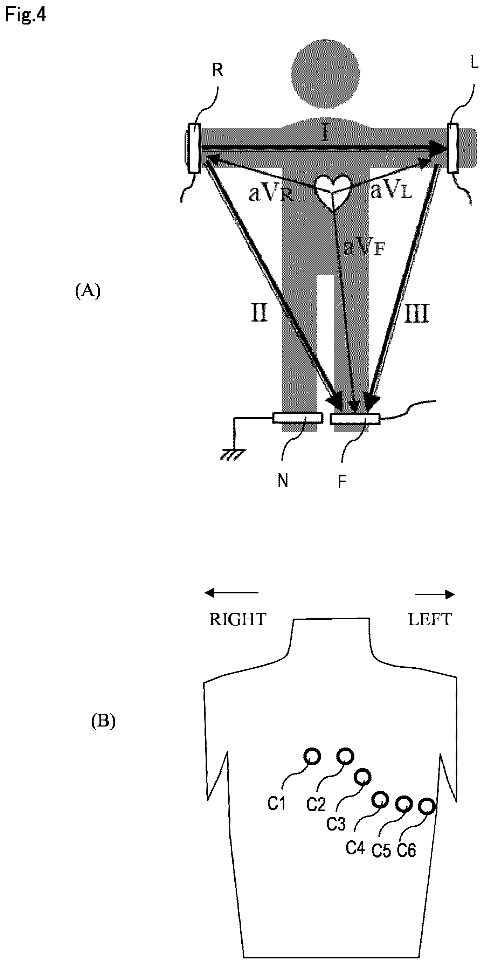

FIG. 4 shows attachment positions of ten (n=10) electrodes R, L, F, N, C1, C2, C3, C4, C5, C6 for the standard 12-lead electrocardiogram. As shown in FIG. 4(A), the electrode R is attached to the right arm. The electrode L is attached to the left arm. The electrode F is attached to the left leg. The electrode N is attached to the right leg. As shown in FIG. 4(A), electrocardiograms (second biological signal) of a total of six channels, i.e., lead I, lead II, lead III, lead aV_{R}, lead aV_{L}, lead aV_{F}, can be obtained by the four limb electrodes R, L, F, N.

As shown in FIG. 4(B), the electrodes C1, C2, C2, C3, C4, C5, C6 are attached to the chest. The electrode C1 is attached at the right sternal border of the fourth intercostal space, and is used to obtain an electrocardiogram of lead V1. The electrode C2 is attached at the left sternal border in the fourth intercostal space, and is used to obtain an electrocardiogram of lead V2. The electrode C3 is attached at a point between the electrode C2 and the electrode C4, and is used to obtain an electrocardiogram of lead V3. The electrode C4 is attached at the intersection of the fourth intercostal space and the left midclavicular line, and is used to obtain an electrocardiogram of lead V4. The electrode C5 is attached at the intersection of the horizontal line at the same height as the electrode C4 and the left anterior axillary line, and is used to obtain an electrocardiogram of lead V5. The electrode C6 is attached at the intersection of the horizontal line at the same height as the electrode C4 and the left midaxillary line, and is used to obtain an electrocardiogram of lead V6.

Thus, electrocardiograms (second biological signal) of a total of six channels, i.e., lead V1, lead V2, lead V3, lead V4, lead V5, lead V6, can be obtained by the six electrodes C1, C2, C2, C3, C4, C5, C6.

Therefore, as the standard 12-lead electrocardiogram, electrocardiograms (second biological signal) of a total of 12 channels, i.e., six channels according to the four limb electrodes R, L, F, N, and six channels according to the six chest electrodes C1, C2, C2, C3, C4, C5, C6, can be obtained.

The ten (n=10) electrodes R, L, F, N, C1, C2, C3, C4, C5, C6 for the standard 12-lead electrocardiogram are each separated, and the electrodes need to be accurately attached at predetermined positions, respectively. When the electrodes are not accurately attached at the predetermined positions, respectively, the obtained electrocardiograms are useless for diagnosis. However, attaching these ten electrodes R, L, F, N, C1, C2, C3, C4, C5, C6 at accurate positions is not easy even for a health care professional in some cases. When there is no health care professional who is well-experienced in attachment of the electrodes, measurement of the electrocardiograms cannot be performed in some cases.

However, when the system 1 of the embodiment is used, the second biological signal corresponding to the standard 12-lead electrocardiogram can be obtained based on the first biological signal obtained by the electrode device 10. Therefore, burden of attaching the electrodes is reduced.

With reference back to FIG. 3(A), in the electrode device 10 of the embodiment, at least one electrode, preferably the multiple electrodes E0 to E15, is attached at the chest center so as to overlap the position of the heart. Here, the arrangement of the electrodes E0 to E15 when the electrode device 10 is attached to the living body is referred to as "first arrangement". FIG. 3(A) shows an example of the first arrangement. The first arrangement shown in FIG. 3(A) is an electrode arrangement in which 16 electrodes E0 to E15 having been integrated are attached at the chest center.

In addition, an electrode arrangement different from the first arrangement will be referred to as "second arrangement". It is sufficient that the second arrangement is different from the first arrangement in at least one of the number of electrodes and the attachment position. The electrode arrangement for the standard 12-lead electrocardiogram shown in FIG. 4 is an example of the second arrangement. The second arrangement shown in FIG. 4 is different from the first arrangement shown in FIG. 3 in both of the number of electrodes and the attachment position. In the second arrangement, the electrodes are attached to a wider area as compared with the first arrangement in which the electrodes are concentrated at the chest center.

In the electrode device 10 shown in FIG. 1 and FIG. 3, m indicating the number of the electrodes E0 to E15 is 16. That is, the electrode device 10 shown in FIG. 1 includes 16 electrodes E0 to E15. These electrodes E0 can include one or more reference electrodes E15. The reference electrode E15 is an electrode for obtaining a living body surface potential (reference potential) to serve as a reference. In the shown electrode device 10, the electrode E15 at the left corner is the reference electrode. Of the m electrodes E0 to E15, the electrodes E0 to E14 other than the reference electrode E15 are measurement electrodes for measuring a p-channel biological signal (living body surface potential) to be provided to the signal processing device 20. Here, the 15 measurement electrodes E0 to E14 are used to measure the body surface potential (first biological signal) in 15 channels based on the reference potential. The potential measured by the measurement electrode E0 to E14 may be a body surface potential based on another measurement electrode E0 to E14. When the body surface potential based on another measurement electrode E0 to E14 is also measured, even though the number of electrodes is the same, the first biological signal (living body surface potential) of a larger number of channels can be obtained.

Thus, the shown electrode device 10 has, as an example, 16 (m=16) electrodes, and can measure the first biological signal of 15 (p=15) channels.

In the electrode device 10 shown in FIG. 1 and FIG. 3, the electrodes E0 to E15 are arranged on a two-dimensional array on the sheet 11. More specifically, the 16 electrodes E0 to E15 are in a two-dimensional array arrangement of four columns vertically and four rows horizontally. The arrangement of the electrodes E0 to E15 is not limited to the two-dimensional array arrangement, and may be another arrangement.

Preferably, the number m of the electrodes E0 to E15 included in the electrode device 10 is at least 3 or more in order to obtain a biological signal of multiple channels. The number m of the electrodes E0 to E15 included in the electrode device 10 is more preferably 9 or more and 25 or less, and further preferably 11 or more and 20 or less.

The electrodes E0 to E15 included in the electrode device 10 are preferably integrated in order to make the electrode device 10 compact or facilitate handling of the electrode device 10. For example, it is preferable that all the electrodes E0 to E15 are integrated so as to be positioned in a reference area X having a predetermined size.

Here, the reference area X is a square area having a size that allows all the electrodes E0 to E15 to be positioned therein, for example. The length of one side of the square forming this reference area X is preferably 30 cm or less. If all the electrodes E0 to E15 are integrated so as to be accommodated in a square whose one side is about 30 cm, it becomes easy to attach the electrodes E0 to E15 to the chest of a person.

The smaller the length of one side of the square forming the reference area X is, the more preferable. For example, the length of one side is more preferably 25 cm or less, more preferably 20 cm or less, and more preferably 15 cm or less. The smaller the length of one side is, the higher the integration degree of the electrodes becomes.

When the length of one side of the square forming the reference area X is too small, formation of the electrodes E0 to E15 or of a wiring described later may become difficult. Therefore, the length of one side is more preferably 5 cm or more, and more preferably 10 cm or more, for example.

In the embodiment, the first biological signal is acquired with respect to multiple channels. That is, a number of channels p of the first biological signal is preferably an integer of 2 or larger. The larger the number of channels p of the first biological signal is, the larger the amount of information necessary for reconstruction of the second biological signal described later becomes, which is preferable.

n indicating the number of the electrodes R, L, F, N, C1, C2, C3, C4, C5, C6 shown in FIG. 4 is 10, and q indicating the number of channels of the second biological signal obtained by these electrodes is 12. That is, in measurement for the standard 12-lead electrocardiogram, ten (n=10) electrodes are used, and the second biological signal of 12 (q=12) channels is measured. In measurement for the standard 12-lead electrocardiogram or in measurement of another second biological signal, n is preferably an integer of 3 or larger, and q is preferably an integer of 2 or larger.

Preferably, the number of channels p obtained in the first arrangement is at about the same level as the number of channels q obtained in the second arrangement, or is a value larger than the number of channels q. When the number of channels p in the first arrangement is larger than the number of channels q in the second arrangement, a large number of signals can be acquired in the first arrangement, and thus, reconstruction of the second biological signal can be accurately performed. For example, when the second arrangement is the electrode arrangement for the standard 12-lead electrocardiogram, the number of channels q can be 12, or less than 12. When q is 12, it is preferable that the number of channels p in the first arrangement using the electrode device 10 is 10, 11, 12, or larger so as to be at least at about the same level as q. When the number of channels p in the first arrangement using the electrode device 10 is set to be 13 or larger, the number of channels p becomes larger than the number of channels q, which is preferable. The number of channels p is more preferably sufficiently larger than the number of channels q, and is, for example, preferably 14 or larger, and further preferably 15 or larger.

Preferably, the number of electrodes m in the first arrangement is a value at about the same level as the number of electrodes n in the second arrangement or larger than the number of electrodes n. When the number of electrodes m in the first arrangement is larger than the number of electrodes n in the second arrangement, a large number of signals can be easily acquired in the first arrangement, which is advantageous. For example, when the second arrangement is the electrode arrangement for the standard 12-lead electrocardiogram, the number of electrodes n=10. In this case, the number of electrodes m in the first arrangement using the electrode device 10 is preferably 9, 10, or larger so as to be at least at about the same level as n. When the number of electrodes m in the first arrangement using the electrode device 10 is set to be 11 or larger, the number of electrodes m becomes larger than the number of electrodes n, which is preferable. The number of electrodes m is more preferably sufficiently larger than the number of electrodes n, and is, for example, preferably 12 or larger, and further preferably 15 or larger.

Preferably, in the first arrangement, the electrodes are arranged at a higher density than in the second arrangement, on the surface of the living body.

With reference back to FIG. 1, the first biological signal of the multiple channels acquired from the electrode device 10 in the first arrangement is provided to the signal processing device 20. Using a generative model, the signal processing device 20 generates the second biological signal of the multiple channels from the first biological signal of the multiple channels. Here, from the first biological signal in time series in a predetermined period, the second biological signal in time series in a predetermined period is generated.

The signal processing device 20 includes a communication device 23 for receiving the first biological signal transmitted from the electrode device 10. The communication device 23 is a device for performing short-range wireless communication using Bluetooth (registered trademark) or the like, for example. The signal processing device 20 includes a computer that has a processor 21 and a storage device 22 connected to the processor. The communication device 23 may be provided inside the computer, or may be provided outside the computer.

The processor 21 is a CPU, for example. The storage device 22 includes a primary storage device and a secondary storage device, for example. The primary storage device is a RAM, for example. The secondary storage device is a hard disk drive (HDD) or a solid-state drive (SSD), for example. The storage device 22 includes a computer program 22c that is executed by the processor 21.

The processor 21 reads out the computer program 22c stored in the storage device 22 and executes the computer program 22c. The computer program 22c in the storage device 22 has program codes including commands for causing a computer to operate as the signal processing device 20. Operation of the signal processing device 20 includes executing a process of: acquiring the first biological signal of the p channels from the m electrodes; inputting the acquired first biological signal of the P channels into a generative model; and outputting the second biological signal of the q channels, from the generative model.

The storage device 22 has a storage region 22a for saving the acquired first biological signal of the p channels. The storage device 22 has a storage region 22b for saving the generated first biological signal of the q channels.

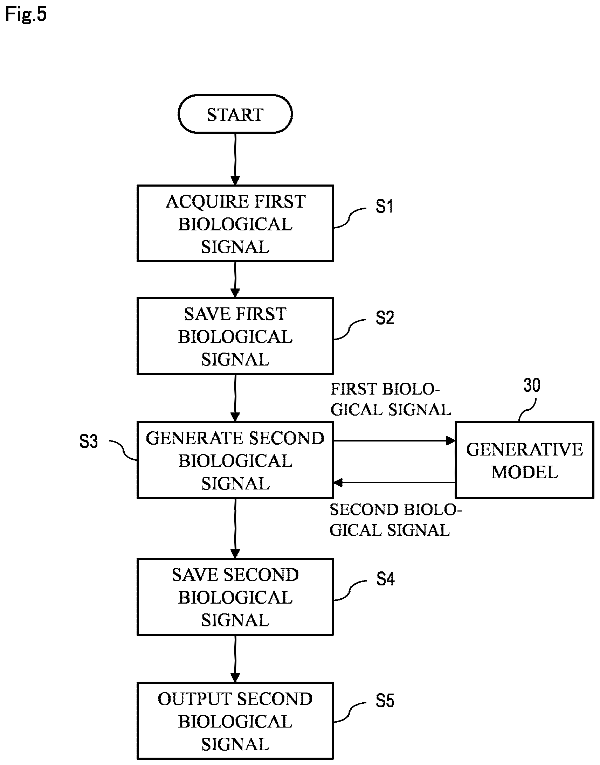

FIG. 5 shows a procedure of a process executed by the signal processing device 20. In the process shown in FIG. 5, from the first biological signal acquired from the electrodes E0 to E15 in the first arrangement, the second biological signal to be obtained by the electrodes in the second arrangement different from the first arrangement is generated. That is, in the process shown in FIG. 5, from the first biological signal actually measured, the second biological signal necessary for diagnosis is reconstructed.

First, in step S1, the signal processing device 20 acquires the first biological signal. For example, the signal processing device 20 receives the first biological signal of the p channels from the electrode device 10 through wireless communication via the communication device 23. In step S2, the signal processing device 20 saves the received first biological signal into the storage device 22.

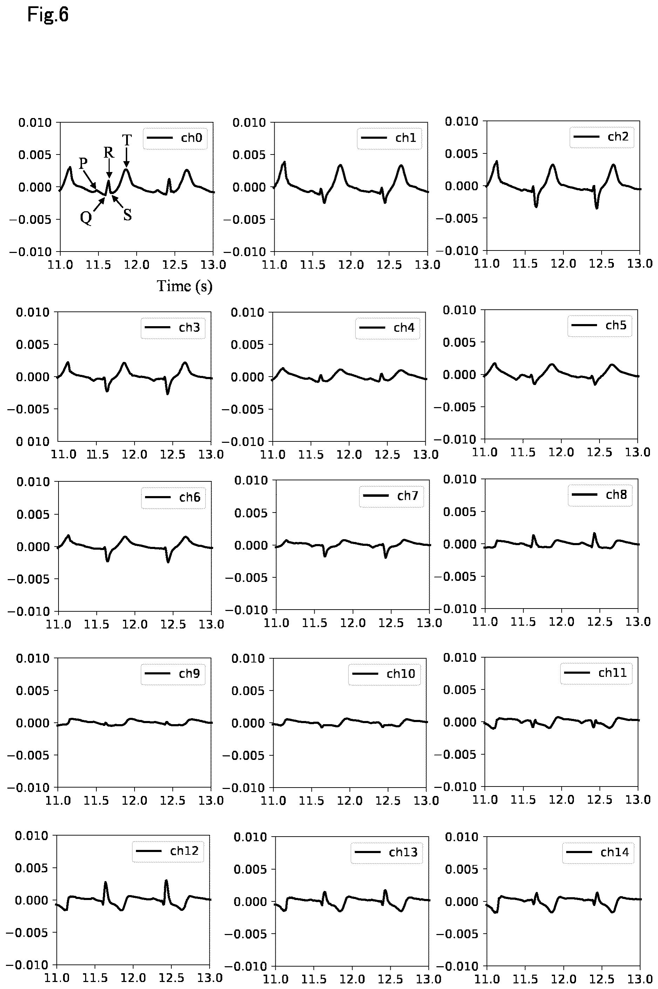

FIG. 6 shows time series data (signal waveform) of the first biological signal (living body surface potential) of the 15 (p=15) channels measured by the 16 (m=16) electrodes E0 to E15. In FIG. 6, the waveforms of the first biological signals of a total of 15 channels, i.e., from ch 0 to ch 14, are shown. In the channels from ch 0 to ch 14, potentials at the electrode E0 to the electrode E14 measured based on the potential of the reference electrode E15 serving as the reference potential are respectively shown.

In each signal waveform, the vertical axis represents potential, and the horizontal axis represents time. Each signal waveform was measured at the same time, and in FIG. 6, the waveform in the same period from 11.0 [s] to 13.0 [s] is shown. As a reference, the positions of P wave, R wave, T wave, Q wave, and S wave in the electrocardiogram are shown together with the signal waveform of ch 0.

The first biological signals of 15 channels shown in FIG. 6 show the potentials at the respective positions of the electrodes E0 to E14 integrally arranged at a high density at the chest center of a person. In the following, as an example, using the first biological signals of these 15 (p=15) channels, electrocardiograms (second biological signal) of 8 (q=8) channels included in a standard 12-lead electrocardiogram are generated. The electrocardiograms of the 8 channels that are generated are of lead I, lead II, lead V1, lead V2, lead V3, lead V4, lead V5, and lead V6, as an example. Those of lead III, lead aV_{R}, lead aV_{L}, and lead aV_{F} may also be generated.

In step S3 in FIG. 5, using a generative model 30, from the time series data of the first biological signal of the p channels in the same period, the signal processing device 20 generates the second biological signal of the q channels in the period.

The generative model 30 is configured to output the second biological signal of the q channels when the first biological signal of the p channels has been inputted. As an example, the generative model 30 is a learned model 30 that has been subjected to machine learning so as to output the second biological signal of the q channels when the first biological signal of the p channels has been inputted. The machine learning is deep learning, as an example.

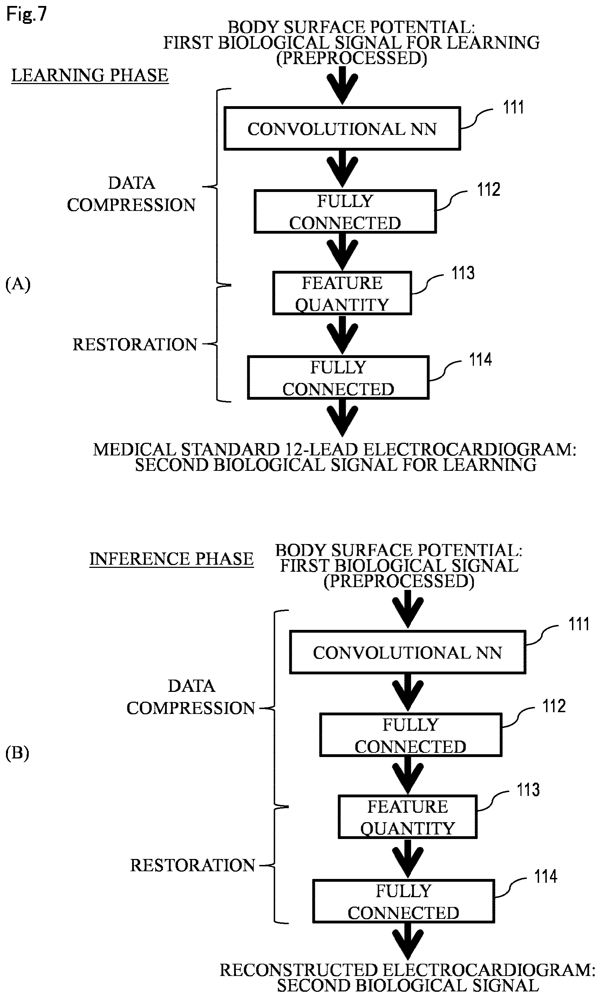

FIG. 7 shows an example of the generative model 30. FIG. 7(A) is a diagram illustrating a machine learning phase of the generative model 30, and FIG. 7(B) is a diagram illustrating an inference phase for reconstructing the second biological signal from the first biological signal. As an example, the generative model 30 includes a convolutional neural network 111 and a fully-connected layer 112, and is configured to obtain a feature quantity 113 of the inputted first biological signal. The generative model 30 reconstructs the second biological signal on the basis of the feature quantity 113 through a fully-connected layer 114, and outputs the resultant second biological signal. The first biological signal that is inputted is subjected to preprocessing such as noise removal in advance, as necessary.

As shown in FIG. 7(A), in the learning phase, using the first biological signal for learning and the second biological signal for learning, machine learning of the model 30 as a neural network is performed. The first biological signal for learning is, as an example, electrocardiograms of the 15 (p=15) channels (see FIG. 6) acquired from the 16 (m=16) electrodes E0 to E15 included in the electrode device 10 affixed at the chest center of a person. The second biological signal for learning is, as an example, electrocardiograms of the 8 (q=8) channels acquired from the 10 (n=10) electrodes for measurement of a standard 12-lead electrocardiogram. The electrocardiograms of the 8 channels are of lead I, lead II, lead V1, lead V2, lead V3, lead V4, lead V5, and lead V6.

In the learning phase, the first biological signal for learning and the second biological signal for learning in the same period, which have been simultaneously measured with respect to the same person, are paired. Machine learning of the model 30 is performed such that, when the first biological signal for learning has been inputted to the model 30, the second biological signal for learning is reconstructed.

In the machine learning, pairs of the first biological signal for learning and the second biological signal for learning of a plurality of persons are used. By using the signals for learning of a plurality of different persons, the model 30 can accurately generate the second biological signal irrespective of individual differences.

Variation can occur in the position of affixing and the angle of affixing the electrode device 10 to the human body. However, by performing machine learning of the model 30 using a large number of signals for learning including such variation, it is possible to accurately generate the second biological signal even when the affixing position and the affixing angle are a little inappropriate. Therefore, affixing of the electrode device 10 of the embodiment may be performed with a little inaccuracy, and is easy as compared with affixing of electrodes for a standard 12-lead electrocardiogram. As a result, naturally, a health care professional can easily affix the electrode device 10, and the patient to whom the electrode device 10 is to be affixed can also easily affix the electrode device 10 himself/herself. Therefore, the electrode device 10 of the embodiment can be used for measurement of an electrocardiogram outside a medical institution, such as at home or the like. Therefore, the electrode device 10 of the embodiment can be utilized in telemedicine and the like.

As shown in FIG. 7(B), in the inference phase (corresponding to step S3 in FIG. 5), the first biological signal is inputted to the model 30. The model 30 outputs the second biological signal reconstructed from the first biological signal. As an example, the first biological signal inputted in the inference phase is electrocardiograms of the 15 (p=15) channels acquired from the 16 (m=16) electrodes E0 to E15 included in the electrode device 10 affixed at the chest center of a person (see FIG. 6). As an example, the second biological signal that is reconstructed is electrocardiograms of the 8 (q=8) channels in the standard 12-lead electrocardiogram. The electrocardiograms of the 8 channels are of lead I, lead II, lead V1, lead V2, lead V3, lead V4, lead V5, and lead V6.

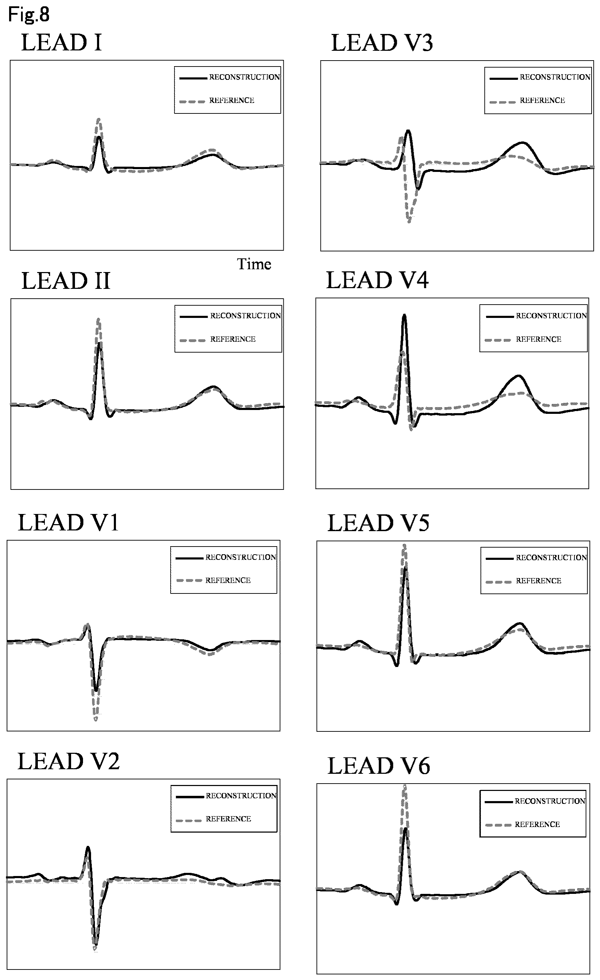

FIG. 8 shows the second biological signal (electrocardiograms) of the 8 channels generated by using the generative model 30 having been subjected to machine learning by using the first biological signal for learning and the second biological signal for learning corresponding to four persons. For generation of the second biological signal, time series data in a predetermined period of the first biological signal of the 15 channels obtained from the electrode device 10 affixed at the chest center of the subject is inputted to the generative model 30. Preferably, the predetermined period has a time length of one heartbeat or more of the heart. The subject is a person different from the four persons from whom the signals for learning were measured.

In each electrocardiogram in FIG. 8, the vertical axis represents potential and the horizontal axis represents time. In FIG. 8, the waveform of "reconstruction" indicated by a solid line is a waveform reconstructed by the generative model 30. The waveform of "reference" indicated by a dotted line in FIG. 8 is a reference signal measured at the same time with the first biological signal, with the electrodes for a standard 12-lead electrocardiogram affixed to the subject. It can be said that the more the waveform of "reconstruction" is approximate to that of "reference", the more accurate the reconstruction is. As shown in FIG. 8, the waveform of "reconstruction" is approximate to that of "reference", and the reconstruction has been accurately performed. In addition, the waveform of "reconstruction" shown in FIG. 8 has been obtained by using the model 30 having been subjected to machine learning by using signals for learning corresponding to four persons. Therefore, it is possible to expect that the accuracy of reconstruction of the second biological signal is further enhanced by using the model 30 having been subjected to machine learning by using a larger number of signals for learning.

With reference back to FIG. 5, in step S4, the signal processing device 20 saves the generated second biological signal into the storage device 22. The signal processing device 20 can output the generated second biological signal to the outside (step S5). Examples of outputting to the outside include displaying on a display, printing by a printer, and transmission to a network. The second biological signal outputted to the outside is used in diagnosis performed by a doctor, for example. According to the present embodiment, even if the electrodes for a standard 12-lead electrocardiogram are not accurately attached to the patient, electrocardiograms included in the standard 12-lead electrocardiogram can be obtained, which is advantageous.

The device/system according to the embodiment is not limited to measurement/generation of an electrocardiogram, and can be used for measurement/generation of another biological signal such as a skeletal muscle electromyogram or a visceral smooth muscle electromyogram.

The present invention is not limited to the above embodiment, and various modifications can be made.

### REFERENCE SIGNS LIST

1 biological signal processing system
10 electrode device
11 sheet
11a first surface
11b second surface
12 wiring
15 communication device
20 signal processing device
21 processor
22 storage device
22a storage region
22b storage region
22c computer program
23 communication device
30 generative model
111 convolutional neural network
112 fully-connected layer
113 feature quantity
114 fully-connected layer
C1 chest electrode
C2 chest electrode
C3 chest electrode
C4 chest electrode
C5 chest electrode
C6 chest electrode
E0 electrode
E1 electrode
E2 electrode
E3 electrode
E4 electrode
E5 electrode
E6 electrode
E7 electrode
E8 electrode
E9 electrode
E10 electrode
E11 electrode
E12 electrode
E13 electrode
E14 electrode
E15 electrode
F limb electrode
H heart
L limb electrode
N limb electrode
R limb electrode
X reference area
m number of electrodes
n number of electrodes
p number of channels
q number of channels

## Claims

1. A biological signal processing system comprising:
m (m is an integer of 3 or larger) electrodes attached in a first arrangement to a living body; and
a signal processing device configured to execute a process of inputting a first biological signal of p (p is an integer of 2 or larger) channels acquired from the m electrodes, into a generative model, and of outputting a second biological signal of q (q is an integer of 2 or larger) channels, from the generative model, wherein
the second biological signal of the q channels is a signal corresponding to signals obtained from n (n is an integer of 3 or larger) electrodes attached in a second arrangement to the living body,
the second arrangement is an electrode arrangement different from the first arrangement in at least one of the number of electrodes and an attachment position, and
the generative model is configured to output the second biological signal of the q channels when the first biological signal of the p channels has been inputted.

2. The biological signal processing system according to claim 1, wherein
the m electrodes attached in the first arrangement to the living body are provided on a single base in a state where a relative positional relationship between the m electrodes is fixed.

3. The biological signal processing system according to claim 1, wherein
the generative model is a learned model having been subjected to machine learning so as to output the second biological signal of the q channels when the first biological signal of the p channels has been inputted.

4. The biological signal processing system according to claim 1, wherein
the second biological signal of the q channels is a signal of 12 channels in a standard 12-lead electrocardiogram or a signal of less than 12 channels included in the standard 12-lead electrocardiogram.

5. The biological signal processing system according to claim 1, wherein
the m electrodes from which the first biological signal of the p channels is acquired are all attached at a chest center of the living body.

6. A signal processing device configured to execute signal processing,
the signal processing comprising:
acquiring a first biological signal of p (p is an integer of 2 or larger) channels from m (m is an integer of 3 or larger) electrodes attached in a first arrangement to a living body; and
inputting the first biological signal of the p channels into a generative model, and outputting a second biological signal of q (q is an integer of 2 or larger) channels, from the generative model, wherein
the second biological signal of the q channels is a signal corresponding to signals obtained from n (n is an integer of 3 or larger) electrodes attached in a second arrangement to the living body,
the second arrangement is an electrode arrangement different from the first arrangement in at least one of the number of electrodes and an attachment position, and
the generative model is configured to output the second biological signal of the q channels when the first biological signal of the p channels has been inputted.

7. A biological signal generation method comprising:
acquiring a first biological signal of p (p is an integer of 2 or larger) channels from m (m is an integer of 3 or larger) electrodes attached in a first arrangement to a living body; and
inputting the first biological signal of the p channels into a generative model, and outputting a second biological signal of q (q is an integer of 2 or larger) channels, from the generative model, wherein
the second biological signal of the q channels is a signal corresponding to signals obtained from n (n is an integer of 3 or larger) electrodes attached in a second arrangement to the living body,
the second arrangement is an electrode arrangement different from the first arrangement in at least one of the number of electrodes and an attachment position, and
the generative model is configured to output the second biological signal of the q channels when the first biological signal of the p channels has been inputted.

8. A computer program configured to cause a computer to execute signal processing,
the signal processing comprising:
acquiring a first biological signal of p (p is an integer of 2 or larger) channels from m (m is an integer of 3 or larger) electrodes attached in a first arrangement to a living body; and
inputting the first biological signal of the p channels into a generative model, and outputting a second biological signal of q (q is an integer of 2 or larger) channels, from the generative model, wherein
the second biological signal of the q channels is a signal corresponding to signals obtained from n (n is an integer of 3 or larger) electrodes attached in a second arrangement to the living body,
the second arrangement is an electrode arrangement different from the first arrangement in at least one of the number of electrodes and an attachment position, and
the generative model is configured to output the second biological signal of the q channels when the first biological signal of the p channels has been inputted.
